# EUROPEAN PATENT SPECIFICATION

(11) **EP 2 485 781 B1**
(45) Date of publication and mention of the grant of the patent: **25.02.2015**
(21) Application number: 10769044.8
(22) Date of filing: 27.09.2010
(51) Int. Cl.: A61M 1/00

(54) **MEDICAL VACUUM ASPIRATION DEVICE**
MEDIZINISCHE VAKUUMASPIRATIONSVORRICHTUNG
DISPOSITIF MÉDICAL D'ASPIRATION SOUS VIDE

(30) Priority: 06.10.2009 GB 0917447
(43) Date of publication of application: 15.08.2012
(73) Proprietor: Marie Stopes International, Fitzroy Square, London W1T 6LP (GB)
(72) Inventor: BLACK, Timothy, Reuben, Ladbrooke, Horsham Sussex RH13 6PX (GB); BROOKE, Gerald, Stroud Gloucestershire GL5 3TJ (GB)
(74) Representative: Richardson, Lyndsey Ann
(86) International application number: PCT/GB2010/051610
(87) International publication number: WO 2011/042719

(56) References cited:
- WO-A1-2005/056080
- WO-A1-2005/097239
- DE-U1- 20 310 058
- US-A- 4 187 849
- US-A1- 2004 193 045
- US-A1- 2008 086 080

## Description

This invention relates to medical vacuum aspiration type apparatus, and in particular, but not exclusively, to such apparatus which is manually operable for use in uterine aspiration or evacuation, for such purposes as abortion or endometrial biopsy.

Medical apparatus of the type described above is disclosed in detail in a patent application numbered WO2005/056080 (Burton et al). In that document, a vacuum is generated in a cylinder by a manually driven plunger operable from one end of the cylinder. A quick release value is provided at the other end of the cylinder, to suddenly release the vacuum and effect aspiration into the cylinder when required. In this application, the term vacuum has been used to describe air pressure which is below ambient pressure, as well as being used to describe the absence, partial absence, momentary absence, or momentary partial absence of a gas or fluid in a closed vessel.

Various aspects of the apparatus described in the Burton document mentioned above have been improved by the inventors and are the subject of this application.

The use of manually operable vacuum aspiration apparatus is worldwide, and so a simple affordable apparatus is needed. Apparatus that is complicated to manufacture, clean and sterilise, is less likely to be adopted in poorer countries where low cost and easy maintenance are key factors. If such apparatus is to be used widely, an easily dismantled assembly is required which can be readily cleaned or sterilised, then easily reassembled.

Another significant aspect is ease of use. In poorer countries, the chance of a less experienced health care worker performing therapy on a patient is greater, and the chances of poor working conditions increases also. So apparatus which is easy to operate and safe to use is very important.

In the Burton document, the valve is formed by closing off a flexible tube which forms an entry nozzle. The entry nozzle has a hole of constant internal diameter which allows only one size of cannula to be inserted therein. Design freedom of the nozzle is restricted because it has to act as an entry nozzle and a closable valve component.

According to a first aspect, the invention provides medical vacuum aspiration apparatus comprising:
a vacuum cylinder substantially closed or closable at a first end by a valve, and closed at a second end by a plunger moveable along the cylinder,
said valve being formed from a flexible nozzle and a releasable nozzle clamp, the nozzle having also an aperture in fluid communication with the chamber, characterised in that said aperture is stepped in diameter or tapered in size to accept cannulae of different sizes in use.

In a refinement, the aperture is stepped or tapered such that the largest diameter or size is closest to the outer end of the nozzle.

In an embodiment the outer end of the nozzle includes an area of reduced diameter.

Thus in an embodiment the reduced diameter portion has a rounded formation on its inner surface. Said formation is preferably similar to that of an 'O' ring at its inner edges, for contacting and stabilising a cannula fitted into the aperture.

In an embodiment, the nozzle has an outer region of generally constant diameter at a location adjacent the stepped or tapered aperture.

Further, in the Burton document, the plunger is withdrawn along the cylinder in a direction away from the valve. The plunger needs to be held at the end of its cylinder stroke by a locking mechanism to prevent it from being sucked back into the cylinder by the vacuum. The locking mechanism uses resilient legs on the plunger which are held together in the cylinder and which part when they are free of restriction after being withdrawn from the cylinder. This locking mechanism sometimes needs two hands to operate, if the legs do not find their locking positions, and when they need to be collapsed back into the cylinder for further aspiration steps.

According to a second aspect, the invention provides a medical vacuum aspiration apparatus comprising:
a vacuum cylinder substantially closed or closeable at a first end by a valve, and closed at a second end by a plunger moveable along an axis of the cylinder,
said plunger having a stem and being restrainable against movement toward the valve when positioned adjacent the second end by a locking mechanism, characterised in that the locking mechanism includes an area of reduced cylinder size at the second end and a portion of the stem of the plunger which, when the plunger is positioned adjacent the second end, is engageable with the restricted area by rotation of the plunger and stem about the axis to effect said engagement.

In an embodiment the reduced cylinder size is formed by a cylinder end cap having a slot, and said stem includes a narrowed portion adjacent the plunger, and an enlarged portion being located at least immediately adjacent the narrowed portion, said rotation about said axis allowing said narrowed portion to rotate in the slot, and allowing said enlarged portion to become misaligned with the slot, such that inward movement of the plunger will cause the enlarged portion to engage with the cap.

In an embodiment, the cap extends into the cylinder.

In a refinement, the slot is open at one side to allow the stem to be removed from the slot by movement transverse to the general direction of relative movement between the stem and the cap.

In an embodiment, the stem has a generally circular cross section at its narrowed portion, and at the enlarged portion, includes said circular cross section and a pair of wings extending from the circular cross section.

The invention extends to any combination of the aspects mentioned above and/or their preferred subsidiary features.

The medical vacuum aspiration apparatus according to the aspects above may include a closed loop handle at the end of the stem of the plunger, formed from a moulded first plastics material, and over moulded with a second plastics material softer than the first material.

In a refinement the second plastics material is disposed around the whole of the inner faces of the closed loop, and preferably around at least the majority of the outer faces.

In an embodiment the apparatus is capable of being dismantled into discrete components and the components include indicia to show the relative orientation of the components when reassembled.

The invention can be put into effect in numerous ways, one embodiment only being described below by way of example, with reference to the drawings, wherein:
Figure 1 shows a pictorial view of a medical vacuum aspiration apparatus;
Figure 2 shows a partial section through the apparatus shown in Figure 1; and
Figure 3 shows a nozzle piece suitable for the apparatus shown in Figures 1 and 2.

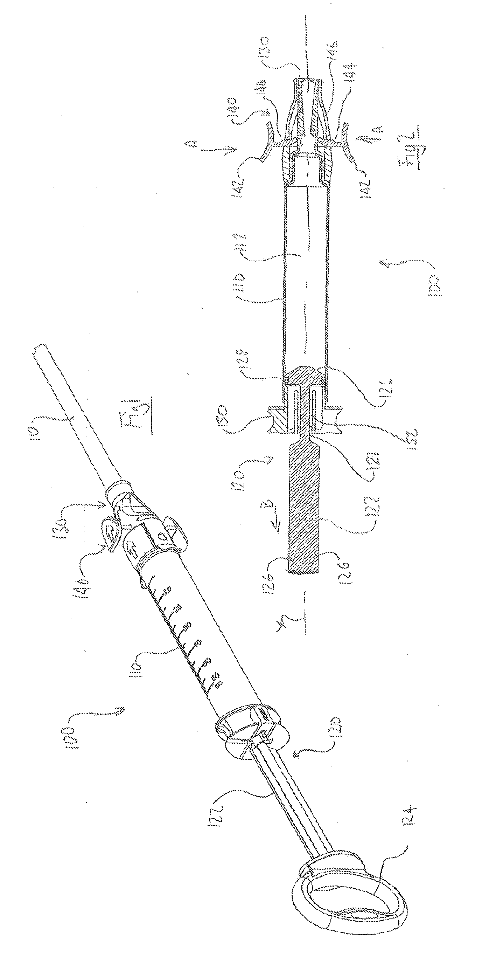

Referring to Figure 1, there is shown a medical vacuum aspiration apparatus 100 suitable for uterine aspiration or evacuation, for example for use in human or animal abortions, endometrial biopsy, and like techniques in which a cannula 10 is used to aspirate intrauterine contents.

The apparatus 100 comprises a vacuum cylinder 110, a plunger 120 (shown partially in this Figure) moveable within the cylinder 110 and including a plunger stem 122 and a plunger handle 124. The plunger may be slid in an end cap 150.

The apparatus 100 includes also a nozzle 130 into which is fitted the cannula 10. The nozzle is manufactured from a flexible material and formed into a tube which is described below in more detail. The nozzle is substantially surrounded by a nozzle clamp 140 which again is described in more detail below. In operation the user closes the clamp components 140 to seal the nozzle 130 at a first end of the cylinder 110 by squeezing the nozzle closed. The user then grasps the vacuum cylinder 110 in one hand and pulls the handle 124. This action produces a reduced pressure within the vacuum cylinder 110 causing the plunger to be dragged back into the cylinder. To prevent this the plunger is locked in place by a mechanism which will be described below. The cannula 10 is manipulated to an area where aspiration is required and the clamp 140 is released so that suction is generated within the cannula 10. The vacuum generated is sufficient to aspirate the area at the end of the cannula 10.

Referring additionally to Figure 2, this drawing shows a section through the apparatus 100 shown in Figure 1, in an approximately vertical plane in the view of Figure 1, through the centre of the apparatus 100. In this view, the plunger 120 is shown more clearly. The plunger 120 includes a piston 126, having an O ring seal 128, for providing a sliding seal. In this view the piston 126 is shown fully withdrawn along the vacuum cylinder 110. In use, prior to the plunger's movement in the direction of arrow B, the nozzle clamp 140 is employed. Two teeth 142 are squeezed into engagement with the flexible nozzle 130 on opposite sides of the nozzle. The nozzle 130, which is described below in more detail, is compressed by the opposing teeth 142 to the point that it is sealed. The nozzle clamp 140 includes also a collar 146, which is a unitary plastics moulding formed completely around the nozzle 130 with no joins along the axis X of the apparatus 100.

In use the teeth 142 are squeezed in the direction of arrow A and are then forced forwardly in a direction away from arrow B. When the teeth 142 are in their clamping positions, pushing them in the direction away from arrow B causes detents 144 to engage with an inner face of the collar 146, so as to hold the teeth in position clamping the nozzle 130. After a vacuum is generated in the cylinder 110, at area 112, then that vacuum can be quickly collapsed, by releasing one or both teeth 142.

At the other, second end of the apparatus 10, in order to generate the vacuum in the area 112, the plunger 120 is withdrawn in the direction of arrow B using the handle 124. It will be noted that the stem 122 of the plunger 120 includes a winged portion including two wings 126, which enlarges its cross sectional shape. The end cap 150 includes a slot 152 which allows the stem to slide in the cap.

When the stem 122 is in position shown in Figure 2, only a narrowed portion 121 of the stem 122 is held in the slot 152. This positioning allows the stem 122 to be rotated about the axis X such that the wings 126 no longer align with the slot 152. The vacuum generated in the area 112 urges the plunger 120 in a direction opposite to arrow B. Thus, the wing portions are forced back towards the end cap 150 and engage with a recess in the cap, to prevent the plunger being urged further back into the vacuum cylinder 110. This arrangement acts as a locking mechanism preventing the plunger 126 from moving back into the chamber 110 while the vacuum is present in the cylinder 110.

In use once the plunger 120 is locked into position the apparatus can be manipulated into place and aspiration therapy can be carried out by unclamping the clamp 140.

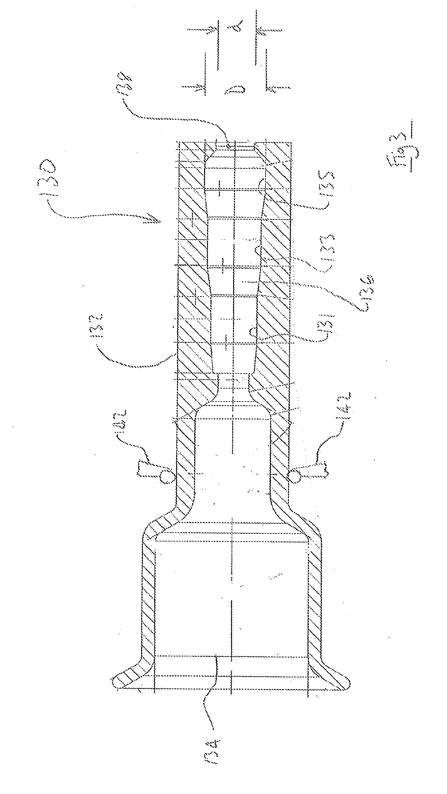

Figure 3 shows an enlarged view of the nozzle 130. The nozzle 130 is manufactured from a moulded flexible silicone material, having an opening 134 for receiving one end of the vacuum cylinder 110 and a further outer diameter 132 around which the components of lamp 140 are located in use. The diameter 132 is constant, for ease of manufacture and for ease of cleaning. In use, the teeth 142 are positioned at a location adjacent the opening 134, for engagement with the constant diameter portion 132.

The nozzle includes an aperture 136 which, in this instance, has three distinct internal diameters 131, 133 and 135. These diameters increase from an initial inner diameter of d to an outer diameter of D, where D ÷ d is in the range of 1.4 - 1.6, more preferably approximately 1.5. This ratio gives a broad range of usability because cannulae of differing sizes can be inserted into the aperture 136.

The aperture 136 includes also a portion 138 of a reduced diameter at the entry to the nozzle. The purpose of the reduced diameter 138 is to act as a steady for a cannula inserted into the aperture 136. The reduced diameter portion 138 has a rounded inner diameter which forms a good seal around the cannula and improves aspiration efficiency also.

One embodiment only of the invention has been illustrated, however various modifications, alterations or improvements, are envisaged, within the scope of the invention. For example, the cylindrical parts used could be replaced by any tubular formations, and the word cylindrical etc is not intended to be limited to circular cross sections. Although plastics and polymer constructions are preferred, other materials or combinations of materials, for example metals or ceramics could be employed also. In Figure 3 a stepped diameter aperture 136 is shown, although, an inwardly tapering aperture could be employed also with equal effect. The apparatus and technique for locking the plunger 120 against movement into the cylinder 110 is a 'twist and lock' arrangement and other twist and lock arrangements are envisaged. The apparatus is intended to be easy to dismantle and to reassemble after cleaning. To aid reassembly, indicia may be provided on the parts to show their relative orientations for reassembly. The handle 124 shown is intended to be formed from a hard plastics material over-moulded with a softer rubber cushion around the inner diameter of its closed loop and at least partially around the outer diameter of the loop.

## Claims

1. Medical vacuum aspiration apparatus (100) comprising:
a vacuum cylinder (110) substantially closed or closable at a first end by a valve, and closed at a second end by a plunger (120) moveable along the cylinder (110),
said valve being formed from a flexible nozzle (130) and a releasable nozzle clamp (140), the nozzle having also an aperture (136) in fluid communication with the cylinder, **characterised in that** said aperture (136) is stepped in diameter or tapered in size to accept cannulae (10) of different sizes in use.

2. Apparatus as claimed in claim 1, wherein the aperture (136) is stepped or tapered such that the largest diameter or size is closest to the outer end of the nozzle (130).

3. Apparatus as claimed in claim 2, wherein the outer end of the nozzle (130) includes an area of reduced diameter (138).

4. Apparatus as claimed in claim 3, wherein the reduced diameter portion (138) has a rounded formation on its inner surface.

5. Apparatus as claimed in claim 4, wherein said formation is similar to that of an 'O' ring at its inner edges, for contacting and stabilising a cannula (10) fitted into the aperture (136).

6. Apparatus as claimed in any one of the preceding claims, wherein the nozzle (130) has an outer region of generally constant diameter (132) at a location adjacent the stepped or tapered aperture (136).

7. Apparatus, as claimed in any of the preceding claims further comprising:
said plunger (120) having a stem (122) and being restrainable against movement toward the valve when positioned adjacent the second end by a locking mechanism, **characterised in that** the locking mechanism includes an area of reduced cylinder size at the second end and a portion of the stem (122) of the plunger which, when the plunger is positioned adjacent the second end, is engageable with the restricted area by rotation of the plunger and stem about the axis to effect said engagement.

8. Apparatus as claimed in claim 7, wherein the reduced cylinder size is formed by a cylinder end cap (150) having a slot (152), and said stem includes a narrowed portion (121) adjacent the plunger (120), and an enlarged portion being located at least immediately adjacent the narrowed portion, said rotation about said axis allowing said narrowed portion (121) to rotate in the slot, and allowing said enlarged portion to become misaligned with the slot, such that inward movement of the plunger will cause the enlarged portion to engage with the cap (150).

9. Apparatus as claimed in claim 8, wherein the cap (150) extends into the cylinder (110).

10. Apparatus as claimed in claim 8 or 9, wherein the slot (152) is open at one side to allow the stem (122) to be removed from the slot (152) by movement transverse to the general direction of relative movement between the stem (122) and the cap (150).

11. Apparatus as claimed in any one of the preceding claims 7 to 10, wherein the stem (122) has a generally circular cross section at its narrowed portion (121), and at the enlarged portion, includes said circular cross section and a pair of wings (126) extending from the circular cross section.

12. Apparatus as claimed in any one of the preceding claims, further including a closed loop handle (124) at the end of the stem (122) of the plunger (120), formed from a moulded first plastics material, and over moulded with a second plastics material softer than the first material.

13. Apparatus as claimed in claim 12, wherein the second plastics material is disposed around the whole of the inner faces of the closed loop, and preferably around at least the majority of the outer faces.

14. Apparatus as claimed in any one of the preceding claims wherein the apparatus is capable of being dismantled into discrete components and the components include indicia to show the relative orientation of the components when reassembled.

## Patentansprüche

1. Medizinische Vakuumsaug-Vorrichtung (100), umfassend einen Vakuumzylinder (110), der an einem ersten Ende durch ein Ventil im wesentlichen geschlossen oder schließbar ist und an einem zweiten Ende durch einen Kolben (120) verschlossen ist, welcher entlang dem Zylinder (110) bewegbar ist, wobei das genannte Ventil von einer flexiblen Düse (130) und einer lösbaren Düsenklemm-Vorrichtung (140) gebildet wird und die Düse auch eine Öffnung (136) aufweist, die mit dem Zylinder in Fluidverbindung steht, **dadurch gekennzeichnet, daß** die Öffnung (136) im Durchmesser abgestuft oder in der Größe abgeschrägt ist, um im Gebrauch Kanülen (10) unterschiedlicher Größen aufzunehmen.

2. Vorrichtung nach Anspruch 1, **dadurch gekennzeichnet, daß** die Öffnung (136) so gestuft oder abgeschrägt ist, daß der größte Durchmesser oder die größte Größe bis zu dem äußeren Ende der Düse (130) geschlossen wird.

3. Vorrichtung nach Anspruch 2, **dadurch gekennzeichnet, daß** das äußere Ende der Düse (130) einen reduzierten Durchmesserbereich (138) aufweist.

4. Vorrichtung nach Anspruch 3, **dadurch gekennzeichnet, daß** der reduzierte Durchmesserbereich (138) auf seiner inneren Oberfläche eine abgerundete Form hat.

5. Vorrichtung nach Anspruch 4, **dadurch gekennzeichnet, daß** die Form ähnlich derjenigen eines O-Rings an seinen inneren Rändern ist, um mit einer Kanüle (10), die in die Öffnung (136) gesteckt ist, in Berührung zu treten und sie zu stabilisieren.

6. Vorrichtung nach einem der vorhergehenden Ansprüche, **dadurch gekennzeichnet, daß** die Düse (130) einen äußeren Bereich von im allgemeinen konstantem Durchmesser (132) an einer Stelle neben der abgestuften oder abgeschrägten Öffnung (136) aufweist.

7. Vorrichtung nach einem der vorhergehenden Ansprüche, wobei der Kolben (120) einen Schaft (122) aufweist und gegen Bewegung zu dem Ventil durch einen Verriegelungsmechanismus hemmbar ist, wenn er sich neben dem zweiten Ende befindet, **dadurch gekennzeichnet, daß** der Verriegelungsmechanismus einen Bereich reduzierter Zylindergröße an dem zweiten Ende aufweist sowie einen Teil des Schaftes (122) des Kolben, der, sobald der Kolben neben dem zweiten Ende angeordnet ist, mit dem verringerten Bereich durch Drehen des Kolbens und des Schaftes um die Achse in Eingriff kommt, um den besagten Eingriff zu bewirken.

8. Vorrichtung nach Anspruch 7, **dadurch gekennzeichnet, daß** die verringerte Zylindergröße durch eine Zylinderendkappe (150) hergestellt wird, die einen Schlitz (152) aufweist, und der Schaft mit einem verengten Teil (121) neben dem Kolben (120) versehen ist, sowie mit einem vergrößerten Teil, der wenigstens unmittelbar neben dem verengten Teil liegt, wobei die Drehung um die genannte Achse ermöglicht, daß der verengte Teil (121) sich in dem Schlitz dreht und dem vergrößerten Teil ermöglicht, mit dem Schlitz außer Fluchtungslage zu kommen, so daß die Einwärtsbewegung des Kolbens bewirkt, daß der vergrößerte Teil mit der Kappe (150) in Berührung tritt.

9. Vorrichtung nach Anspruch 8, **dadurch gekennzeichnet, daß** die Kappe (150) sich in den Zylinder (110) erstreckt.

10. Vorrichtung nach Anspruch 8 oder 9, **dadurch gekennzeichnet, daß** der Schlitz (152) auf der einen Seite offen ist, so daß der Schaft (122) aus dem Schlitz (152) durch Bewegen quer zu der allgemeinen Richtung der
Relativbewegung zwischen dem Schaft (122) und der Kappe (150) entfernt werden kann.

11. Vorrichtung nach einem der vorhergehenden Ansprüche 7 bis 10, **dadurch gekennzeichnet, daß** der Schaft (122) an seinem engsten Teil (121) einen im allgemeinen kreisrunden Querschnitt hat und an dem vergrößerten Teil den kreisrunden Querschnitts sowie ein Paar Flügel (126) aufweist, die sich von dem kreisrunden Querschnitt aus erstrecken.

12. Vorrichtung nach einem der vorhergehenden Ansprüche, ferner **gekennzeichnet durch** einen Handgriff (124) in Form einer geschlossenen Schleife an dem Ende des Schaftes (122) des Kolbens (120), der aus einem gegossenen ersten Kunststoffmaterial besteht und einem Überzug aus einem zweiten Kunststoffmaterial, das weicher ist als das erste Material.

13. Vorrichtung nach Anspruch 12, **dadurch gekennzeichnet, daß** das zweite Kunststoffmaterial rund um die gesamten Innenflächen der geschlossenen Schleife und vorzugsweise rund um wenigstens den Hauptteil der äußeren Flächen angeordnet ist.

14. Vorrichtung nach einem der vorhergehenden Ansprüche, **dadurch gekennzeichnet, daß** die Vorrichtung in bestimmte Komponenten auseinander genommen werden kann und die Komponenten Kennzeichen aufweisen, um die relative Ausrichtung der Komponenten zu zeigen, wenn sie zusammengebaut sind.

## Revendications

1. Appareil medical d'aspiration sous vide (100) comprenant :
un cylindre sous vide (110) sensiblement fermé ou apte à être fermé à une première extrémité par une valve, et fermé à une seconde extrémité par un piston (120) déplaçable le long du cylindre (110),
ladite valve étant formée d'une buse flexible (130) et d'une pince de buse amovible (140), la buse ayant également une ouverture (136) en communication fluidique avec le cylindre, **caractérisé en ce que** ladite ouverture (136) est échelonnée en diamètre ou effilée en dimension pour accepter, lors de l'utilisation, des canules (10) de différentes dimensions.

2. Appareil selon la revendication 1, dans lequel l'ouverture (136) est échelonnée ou effilée de telle sorte que le diamètre le plus grand ou la dimension la plus grande est le ou la plus proche de l'extrémité extérieure de la buse (130).

3. Appareil selon la revendication 2, dans lequel l'extrémité extérieure de la buse (130) comprend une zone de diamètre réduit (138).

4. Appareil selon la revendication 3, dans lequel la partie de diamètre réduit (138) a une formation arrondie sur sa surface intérieure.

5. Appareil selon la revendication 4, dans lequel ladite formation est similaire à celle d'un joint torique à ses bords intérieurs, pour entrer en contact avec une canule (10) montée dans l'ouverture (136) et la stabiliser.

6. Appareil selon l'une quelconque des revendications précédentes, dans lequel la buse (130) a une région extérieure de diamètre généralement constant (132) à un emplacement adjacent à l'ouverture échelonnée ou effilée (136).

7. Appareil selon l'une quelconque des revendications précédentes, comprenant en outre :
ledit piston (120) ayant une tige (122) et étant apte à être restreinte à l'encontre d'un déplacement vers la valve lorsqu'il est positionné de façon adjacente à la seconde extrémité par un mécanisme de verrouillage, **caractérisé en ce que** le mécanisme de verrouillage comprend une zone de dimension de cylindre réduite à la seconde extrémité, et une partie de la tige (122) du piston qui, lorsque le piston est positionné de façon adjacente à la seconde extrémité, est apte à être engagé avec la zone restreinte par rotation du piston et de la tige autour de l'axe pour réaliser ledit engagement.

8. Appareil selon la revendication 7, dans lequel la dimension de cylindre réduite est formée par un capuchon d'extrémité de cylindre (150) ayant une fente (152), et ladite tige comprend une partie rétrécie (121) adjacente au piston (120), et une partie agrandie située au moins immédiatement adjacente à la partie rétrécie, ladite rotation autour dudit axe permettant à ladite partie rétrécie (121) de tourner dans la fente, et permettant à ladite partie agrandie de se désaligner d'avec la fente, de telle sorte qu'un mouvement vers l'intérieur du piston amènera la partie agrandie à s'engager avec le capuchon (150).

9. Appareil selon la revendication 8, dans lequel le capuchon (150) s'étend dans le cylindre (110).

10. Appareil selon la revendication 8 ou 9, dans lequel la fente (52) est ouverte sur un premier côté pour permettre à la tige (122) d'être retirée de la fente (152) par un mouvement transversal à la direction générale de mouvement relatif entre la tige (122) et le capuchon (150).

11. Appareil selon l'une quelconque des revendication 7 à 10, dans lequel la tige (122) a une section transversale généralement circulaire à sa partie rétrécie (121) et, à la partie agrandie, comprend ladite section transversale circulaire et une paire d'ailettes (126) s'étendant à partir de la section transversale circulaire.

12. Appareil selon l'une quelconque des revendications précédentes, comprenant en outre une poignée à boucle fermée (124) à l'extrémité de la tige (122) du piston (120), formée d'une première matière plastique moulée, et surmoulée avec une seconde matière plastique plus souple que la première matière.

13. Appareil selon la revendication 12, dans lequel la seconde matière plastique est disposée autour de la totalité des faces intérieures de la boucle fermée, et, de préférence, autour d'au moins la majorité des faces extérieures.

14. Appareil selon l'une quelconque des revendications précédentes, dans lequel l'appareil est apte à être démonté en composants discrets, et les composants comprennent des indices pour montrer l'orientation relative des composants lorsqu'ils sont ré-assemblés.
